# EUROPEAN PATENT APPLICATION

(11) **EP 2 253 337 A1**
(43) Date of publication of application: **24.11.2010**
(21) Application number: 09160530.3
(22) Date of filing: 18.05.2009
(51) Int. Cl.: A61L 29/00

(54) **Balloon catheter comprising pressure sensitive microcapsules.**

(71) Applicant: Encapson B.V., 6525 AJ Nijmegen (NL)
(72) Inventor: Ayres, Lee, 6525 AJ, NIJMEGEN (NL); Vriezema, Dennis Manuel, 6525 AJ, NIJMEGEN (NL); Hanssen, Johannes Hendrikus Leonardus, 6577 JL, ERLECOM (NL); Opsteen, Johannes Antonius, 6525 AJ, NIJMEGEN (NL)
(74) Representative: Habets, Winand

(57) **Abstract**

The invention provides a solution to the above mentioned problem in that it provides a catheter balloon comprising a flexible coating on its outer surface wherein a plurality of microparticles are contained wherein said coating comprises a material selected from the group consisting of poly(*N*-vinyl-pirrolidone, poly(*N*-vinyl-pirrolidone-co-butylacrylate), poly(4-vinyl pyridine), polyacrylamides, e.g. poly(*N-*isopropylacrylamide), poly(amido-amines), poly(ethylene imine), poly(ethylene oxide-*block*-propylene oxide), poly(ethylene oxide-*block*-propylene oxide-*block*-ethylene oxide), poly(styrene-block-isobutylene-block-styrene), poly(hydroxystyrene-block-isobutylene-*block*-hydroxystyrene), polydialkylsiloxanes, polysaccharides, polyacrylates and polyalkylmethacrylates, e.g. polymethylmethacrylate and poly(2-hydroxyethylmethacrylate) and wherein said microparticles comprise a material selected from the group consisting of polyesters, e.g. poly(lactic acid), poly(lactic-co-glycol acid), poly(glycolic acid), poly(3-hydroxybutyrate), poly(3-hydroxyvalerate), poly(3-hydroxybutyrate-co-3-hydroxyvalerate) and polycaprolactone, polyamides, polysaccharides, polyurethanes, polyalkylmethacrylates and polyacrylates, e.g. polymethylmethacrylate and poly(2-hydroxyethylmethacrylate) and wherein the microparticles comprise a pharmaceutically active compound.

## Description

### Field of the invention

The present invention relates to balloon catheters, more in particular to balloon angioplasty catheters from which plaque reducing compounds can be released.

### Background of the invention

Balloon catheters are currently being used to open up blood vessels that are affected by plaque. It is known in the art that there are drugs that reduce or dissolve plaque. C. Herdeg et al. describe how local delivery of paclitaxel prevents restenosis (J. Am. Coll. Cardiol. 35, 1969-1976 (2000)). The delivery of drugs using a balloon catheter has previously been reported by A. Posa et al. in Coron. Artery Dis. 19, 243-247 (2008).

In US patent application no. 5,893,840 and references therein a description is given of balloon catheters capable of delivering drugs by inflating the balloon in the lumen of a blood vessel. US 5,893,840 describes that microcapsules may be coated on a balloon mounted on a catheter. When the balloon is inflated the microcapsules rupture due to stretching of the coating.

This has the drawback that a part of the therapeutic compound may be flushed away by the blood stream before the balloon blocks the lumen completely. In this way, only a part of the plaque affecting compound is delivered to the desired location.

EP1981559 describes a method to coat folded balloons of balloon catheters for drug delivery.

There remains a need for a device that ensures more efficient and safe delivery of drugs to a local target in the blood vessel.

### Summary of the invention

The invention provides a solution to the above mentioned problem in that it provides a catheter balloon comprising a flexible coating on its outer surface wherein a plurality of microparticles are contained wherein said coating comprises a material selected from the group consisting of poly(*N*-vinyl-pirrolidone), poly(*N*-vinyl-pirrolidone-co-butylacrylate), poly(4-vinyl pyridine), polyacrylamides, e.g. poly(*N-*isopropylacrylamide), poly(amido-amines), poly(ethylene imine), poly(ethylene oxide-*block*-propylene oxide), poly(ethylene oxide-*block*-propylene oxide-*block*-ethylene oxide), poly(styrene-*block*-isobutylene-*block*-styrene), poly(hydroxystyrene-*block-*isobutylene-*block*-hydroxystyrene), polydialkylsiloxanes, polysaccharides, polyacrylates and polyalkylmethacrylates, e.g. polymethylmethacrylate and poly(2-hydroxyethylmethacrylate) and wherein said microparticles comprise a material selected from the group consisting of polyesters, e.g. poly(lactic acid), poly(lactic-*co-*glycol acid), poly(glycolic acid), poly(3-hydroxybutyrate), poly(3-hydroxyvalerate), poly(3-hydroxybutyrate-*co*-3-hydroxyvalerate) and polycaprolactone, polyamides, polysaccharides, polyurethanes, polyalkylmethacrylates and polyacrylates, e.g. polymethylmethacrylate and poly(2-hydroxyethylmethacrylate) and wherein the microparticles comprise a pharmaceutically active compound.

This catheter balloon may be used in a method for delivering a pharmaceutically active compound to a predestinated target in a blood vessel. For that purpose, the device is inserted into a patient in need of such a therapy, monitoring whether the device has reached its predestinated target and inflating the balloon, thereby releasing the pharmaceutically active compound.

### Detailed description of the invention

The invention provides a catheter balloon comprising a coating that contains microparticles encapsulating a pharmaceutically active compound.

The catheter balloon may be any conventional balloon, for instance the balloons that are commercially available from for instance Abbott, Boston Scientific, Cordis and Medtronic.

The microparticle should comprise a material selected from the group consisting of polyesters, e.g. poly(lactic acid), poly(lactic-co-glycol acid), poly(glycolic acid), poly(3-hydroxybutyrate), poly(3-hydroxyvalerate), poly(3-hydroxybutyrate-co-3-hydroxyvalerate) and polycaprolactone, polyamides, polysaccharides, polyurethanes, polyalkylmethacrylates and polyacrylates, e.g. polymethylmethacrylate and poly(2-hydroxyethylmethacrylate). This provides the microparticle with the optimal brittleness so that it will rupture when the balloon is inflated and the microparticles are pressed against the blood vessel wall. Such microparticles, however, will remain intact during the inflation process and will not rupture due to shear stress as is the case with conventional microparticles contained in a conventional coating.

The shape of the microparticles may be spherical, ellipsoid, amorphous, cubical, elongated, truncated, orthorhombic or cylindrical. Their average diameter can range from 0.1 to 100 micrometer, preferably between 0.5 to 20 micrometer. Preferred are spherical hollow microcapsules with a shell that is brittle.

Suitable methods for the preparation of microcapsules are described in WO8808300 by E. Mathiowitz and R.S. Langer in US5,893,840 and in Macromol. Bioscience, 8, 991-1005 (2008) by D. Lensen et al., the disclosures of which are incorporated herein by reference.

The microparticles encapsulate a pharmaceutically active compound or a drug. This may be in the form of a solid, e.g. crystalline, semi-crystalline or amorphous material, a gel, a sol-gel, an oil, suspension, dispersion, emulsion or a solution. The type of drug may be any drug which is beneficial in treating the lumen of a blood vessel. The pharmaceutically active compound may also be a compound used in the diagnosis of a particular disease. Suitable pharmaceutically active compounds or drugs are exemplified in US2009/0054837 which is incorporated herein by reference in its entirety.

The coating of a device according to the invention consists of a material selected from the group consisting of poly(*N*-vinyl-pirrolidone), poly(*N*-vinyl-pirrolidone-*co*-butylacrylate), poly(4-vinyl pyridine), polyacrylamides, e.g. poly(*N-*isopropylacrylamide), poly(amido-amines), poly(ethylene imine), poly(ethylene oxide-*block*-propylene oxide), poly(ethylene oxide-*block*-propylene oxide-*block*-ethylene oxide), poly(styrene-*block*-isobutylene-*block*-styrene), poly(hydroxystyrene-*block-*isobutylene-*block*-hydroxystyrene), polydialkylsiloxanes, polysaccharides, polyacrylates and polyalkylmethacrylates, e.g. polymethylmethacrylate and poly(2-hydroxyethylmethacrylate). The combination of these coating materials with the selected materials for the microparticles provide the device with the unique property that the particles will not release their content unless they are pressed against the wall of the blood vessel.

This selection of materials provides a particularly good hydrophilic and flexible coating which lowers the friction in the lumen and can keep the microparticles adhered while the balloon is moved through the lumen and during inflation.

Suitable methods for applying a coating may be found in WO2007/089761, US 5,893,840 and US 2009/0054837 and in WO0139811 which are incorporated by reference in its entirety herein.

The microparticles can be applied on the balloon in several ways. One approach is to apply a coating on the balloon and the microparticles are applied on the balloon while the coating is still wet. Another approach is to mix the microparticles with the coating solution before it is applied to the balloon. Yet another way is to make the microparticles adhere via electrostatic interactions. This can be achieved by coating the balloon with a charged coating and then add microparticles that are oppositely charged. The microparticles can also be covalently linked to either the surface of the device or the coating, or linked to the device or coating via physisorption or chemisorption.

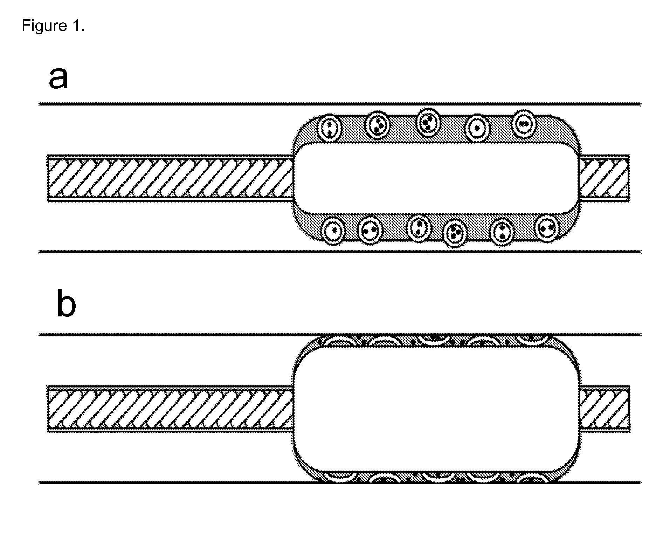

Particularly good results were achieved when a non-rigid coating consisting of poly(N-vinylpirrolidone) was combined with microparticles consisting of poly(methyl urea). Such a combination resulted in a superior performance of the coated microparticles in that the balloon catheter released the drug only when the balloon pressed against a surface upon inflation and not during the inflating process. This is illustrated in figures 1a and 1b.

The combination of the coating materials with the materials for the microparticles as described herein allows for the construction of balloon catheters that are coated with microparticles filled with a therapeutic substance that is released only when pressed against a surface by inflation of the balloon. The coating is flexible enough to stretch with the expanding balloon and does not rupture the microparticles.

The balloon according to the invention may be used in a method for delivering a pharmaceutically active compound to a predestinated target in a blood vessel. For that purpose, the device is inserted into a patient in need of such a therapy, monitoring whether the device has reached its predestinated target and inflating the balloon, thereby releasing the pharmaceutically active compound.

In this way stenosis and plaque formation may effectively be treated.

### Legend to the figures

Figure 1a is a representation of a balloon catheter inside a lumen in the deflated state having a coating with microparticles that contain a drug.
Figure 1b is a representation of a balloon catheter inside a lumen in the inflated state. The microparticles have released the drug when they ruptured by the pressure against the lumen wall.

### Example

Microcapsules of poly( methyl urea) were prepared following the procedure described by E.N. Brown et al. in J. Microencapsulation, 20, 719-730 (2003).

In general, a suitable method for the preparation of poly(methyl urea) microparticles is to dissolve urea (5.0 g, 83 mmol), ammonium chloride (0.5 g, 9.5 mmol) and resorcinol (0.5 g, 4.5 mmol) in a 2,5 % (w/w) solution of poly(ethylene-*alt*maleic anhydride) in water (200 ml). The pH may be raised from 2.44 to 3.70 by dropwise addition of a 0.1 M NaOH solution and, subsequently, lowered to 3.50 using a 0.1 M HCl solution.

The aqueous solution was agitated with an Ultra-Turrax at 15,200 rpm and a few droplets of 1-octanol were added to eliminate foam formation. A slow stream of paraffin containing a minute amount of Oil-Red-O was added to form an emulsion. The high speed stirring with the Ultra-Turrax was continued for 5 minutes in order to stabilize the emulsion.

Afterwards, the emulsion was transferred to a beaker equipped with a magnetic stirring bar and formaldehyde 37 % (11.6 ml, 422 mmol) was added. The reaction mixture was slowly heated (1 °C/min) in a temperature controlled water bath to the target temperature of 55 °C. After 4 hours the magnetic stirring and heating was ended. Once cooled to ambient temperature, the capsules were purified by filtration and washing with distilled water.

The balloon of a Liberte^{tm} MR catheter of Boston Scientific was coated with Labocoat^{®} from Labo. The microcapsules prepared with the method described above were applied to the wet coating by spray drying. The balloon was left to dry at room temperature and atmospheric pressure.

The functioning of the coated balloon catheter was demonstrated by inserting the balloon in a clear Tygon® tube from Saint-Gobain with an inner diameter of 2.4 mm and an outer diameter of 4.0 mm. The balloon was inserted in the tube and fixed on a Zeiss Axiovert 135 TV microscope. The balloon was inflated and when the microcapsules were pressed against the wall of the tube they ruptured and released their content.

## Claims

1. Catheter balloon comprising a flexible coating on its outer surface wherein a plurality of microparticles are contained wherein said coating comprises a material selected from the group consisting of poly(N-vinyl-pirrolidone), poly(N-vinyl-pirrolidone-co-butylacrylate), poly(4-vinyl pyridine), polyacrylamides, e.g. poly(N-isopropylacrylamide), poly(amido-amines), poly(ethylene imine), poly(ethylene oxide-*block*-propylene oxide), poly(ethylene oxide-*block*-propylene oxide-*block*-ethylene oxide), poly(styrene-*block*-isobutylene-*block*-styrene), poly(hydroxystyrene-*block*-isobutylene-*block*-hydroxystyrene), polydialkylsiloxanes, polysaccharides, polyacrylates and polyalkylmethacrylates, e.g. polymethylmethacrylate and poly(2-hydroxyethylmethacrylate) and wherein said microparticles comprise a material selected from the group consisting of polyesters, e.g. poly(lactic acid), poly(lactic-co-glycol acid), poly(glycolic acid), poly(3-hydroxybutyrate), poly(3-hydroxyvalerate), poly(3-hydroxybutyrate-co-3-hydroxyvalerate) and polycaprolactone, polyamides, polysaccharides, polyurethanes, polyalkylmethacrylates and polyacrylates, e.g. polymethylmethacrylate and poly(2-hydroxyethylmethacrylate) and wherein the microparticles comprise a pharmaceutically active compound.

2. Method for delivering a pharmaceutically active compound to a predestinated target in a blood vessel by inserting a device according to claim 1 into a patient in need thereof, monitoring whether the device has reached its predestinated target and inflating the balloon, thereby releasing the pharmaceutically active compound.
